# EUROPEAN PATENT APPLICATION

(11) **EP 0 522 845 A1**
(43) Date of publication of application: **13.01.1993**
(21) Application number: 92306278.0
(22) Date of filing: 08.07.1992
(51) Int. Cl.: A61B 5/083, G01N 33/00

(54) **Method and apparatus for measuring a parameter of a gas in isolation from gas pressure fluctuations**

(30) Priority: 08.07.1991 US 725723
(71) Applicant: PURITAN-BENNETT CORPORATION, Kansas City, Kansas (US)
(72) Inventor: Goulding, Peter, Oceanside, California 92057 (US)
(74) Representative: Mayes, Stuart David

(57) **Abstract**

There is described a system for measuring a parameter of a test gas (31), the system including a source of the test gas, a sensor (11) in fluid communication with the source of the test gas operative to measure the parameter of the test gas, and pump means (13) in fluid communication with the sensor to draw the test gas from the source of the test gas into the sensor, and additionally comprising an air capacitor (15) upstream from the sensor in fluid communication therewith and operative to store a quantity of gas; a source of an alternate gas (17) having an essentially constant pressure; fluid flow control means (19) having a first fluid flow path (53;41) connectable between said air capacitor and said source of the test gas, and a second fluid flow path (37,41) connectable between said air capacitor and said source of alternate gas, the fluid flow control means being operative to open the first fluid flow path for the test gas into the air capacitor, to thereafter close said first flow path and to open the second fluid flow path between the air capacitor and the alternate source and to thereby isolate the air capacitor and the sensor from any fluctuations in the pressure of the test gas; indicator means (51) in electrical communication with said sensor for providing an output signal representing the measure of the unknown parameter; and timing control means (47) in electrical communication with said fluid flow control means and said indicator means for controlling said indicator means to provide said output signal only when the pressure of the test gas in the sensor is held constant.

There is also described a method of measuring an unknown parameter of a test gas in isolation from gas pressure fluctuations comprising the steps of opening a first fluid flow path for the test gas into an air capacitor; drawings the test gas through the air capacitor and into a sensor for measurement of the unknown parameter, thereby filling the air capacitor with the test gas; closing the first fluid flow path; opening a second fluid flow path between a constant pressure alternate gas source and the air capacitor and thereby isolating the sensor from any fluctuations in the pressure of the test gas; and measuring the unknown parameter of the test gas under constant pressure conditions before any substantial mixing of the alternate gas with the test gas stored in the air capacitor occurs.

## Description

### BACKGROUND OF THE INVENTION

### Field of the Invention

The present invention relates generally to the measurement of parameters of gases and more particularly to a method and apparatus for measuring an unknown parameter of a gas in isolation from fluctuations in the pressure of the gas.

### Description of the Related Art

There are numerous instances in which an unknown parameter of a gas must be measured. For example, in the medical field it is often necessary to measure such parameters as oxygen and carbon dioxide concentrations in a gas mixture in either an inspiration or an expiration line of a patient ventilator. Fluctuations in the pressure of the gas during the measurement process can affect the accuracy of the measurements or even render performance of the measurements impossible. Therefore, it is highly desirable to keep the pressure of the gas relatively constant while the measurements are being performed.

If the gas is not in motion, it is relatively easy to keep the pressure constant so as to carry out the necessary measurements. Even if the gas is in motion, if the pressure of the moving gas remains relatively constant, the concentration measurements can be perforved satisfactorily. However, if the gas flow rate and pressure are not uniform, as is the case in an airway of a patient respirator, accurate parameter measurements become diflicult or impossible.

Recent advances in medical diagnostic and therapeutic procedures have created a need for a way to continuously monitor parameters of the gases being inhaled or exhaled by a patient on a respirator. Various methods of measuring such parameters have been proposed, but none of them has adequately solved the problem of making accurate measurements under the conditions of fluctuating pressure and flow rate which are encountered in respirator airways. Therefore, there remains a need for a way to measure parameters of a gas such as a gas in a patient airway in isolation from fluctuations in the pressure and flow rate of the gas.

### SUMMARY OF THE INVENTION

The present invention provides a method and apparatus for measuring a parameter of a test gas in isolation from fluctuations in the pressure and flow rate of the gas by drawing the gas into an air capacitor and then admitting an alternative gas at a constant pressure to an inlet of the capacitor while the measurement is being performed.
According to a first aspect of the present invention there is provided
a system for measuring a parameter of a test gas, the system including a source of the test gas, a sensor in fluid communication with the source of the test gas operative to measure the parameter of the test gas, and pump means in fluid communication with the sensor to draw the test gas from the source of the test gas into the sensor, an improvement for isolating the sensor from any fluctuations in the pressure of the test gas,
characterized by:
an air capacitor upstream from the sensor in fluid communication therewith and operative to store a quantity of gas;
a source of an alternate gas having an essentially constant pressure;
fluid flow control means having a first fluid flow path connectable between said air capacitor and said source of the test gas, and a second fluid flow path connectable between said air capacitor and said source of alternate gas, the fluid flow control means being operative to open the first fluid flow path for the test gas into the air capacitor, to thereafter close said first flow path and to open the second fluid flow path between the air capacitor and the alternate source and to thereby isolate the air capacitor and the sensor from any fluctuations in the pressure of the test gas; indicator means in electrical communication with said sensor for providing an output signal representing the measure of the unknown parameter; and timing control means in electrical communication with said fluid flow control means and said indicator means for controlling said indicator means to provide said output signal only when the pressure of the test gas in the sensor is held constant.

The air capacitor prevents any substantial mixing of the alternate gas with the stored test gas for a sufficient time to enable the sensor to measure the unknown parameter of the test gas under constant pressure conditions. The pressure isolation provided by the air capacitor permits the test gas to flow continuously through the sensor during the measurement process while preventing pressure fluctuations from interfering with accuracy of the measurement.

In a preferred embodiment of the invention, the fluid flow control means comprises a valve, such as a solenoid valve, having a first inlet in fluid communication with a source of the test gas, a second inlet in fluid communication with the alternate gas source and an outlet in fluid communication with the air capacitor. The valve opens the first flow path by connecting the outlet to the first inlet and opens the second flow path by connecting the outlet to the second inlet.

The fluid flow control means preferably also includes timing control means such as a microprocessor or the like which selectively applies power to the solenoid valve to control the flow of gases into the air capacitor. The microprocessor also controls an output display or the like to ensure that the sensor output is provided only during the time that the pressure of the test gas in the sensor is being held constant. In a preferred embodiment, the test gas comprises an inspired gas in an airway of a patient respirator. The alternate gas source preferably comprises the ambient atmosphere.

The air capacitor preferably comprises an elongated chamber characterized by a length-to-diameter ratio much larger than unity; for example, a chamber having a length of about 75 centimeters and a diameter of about 0.5 centimeters has been found to give satisfactory results. The air capacitor can be fabricated, for example, by forming an elongated channel which establishes a fluid flow path between two cavities in a block of material, or can simply comprise a similar length of tubing having a similar diameter, and having appropriate adapters at each end.

According to a second aspect of the invention, a method of measuring an unknown parameter of a test gas in isolation from gas pressure fluctuations is provided that utilizes apparatus of the kind described above. The method comprises the steps of opening a first fluid flow path for the test gas into an air capacitor: drawing the test gas through the air capacitor and into a sensor for measurement of the unknown parameter, thereby filling the air capacitor with the test gas; closing the first fluid flow path; opening a second fluid flow path between a constant pressure alternate gas source and the air capacitor and thereby isolating the sensor from any fluctuations in the pressure of the test gas; and measuring the unknown parameter of the test gas under constant pressure conditions before any substantial mixing of the alternate gas with the test gas stored in the air capacitor occurs. In a preferred embodiment for use in measuring parameters of a mixture of inspired gases in an airway of a patient respirator, the step in the above method of opening the first fluid flow path comprises opening a fluid flow path between the airway and the air capacitor.

An embodiment of the present invention will now be described by way of example with reference to the accompanying drawings in which:

### BRIEF DESCRIPTION OF THE DRAWINGS

FIGURE 1 is a fluid flow diagram of apparatus embodying the invention for measuring a parameter of a gas in isolation from pressure fluctuations;
FIG. 2 is a perspective view of an air capacitor block located upstream of the sensor of the apparatus of Fig. 1;
FIG. 3 is a top plan schematic view of the air capacitor FIG. 2;
FIG. 4 is a top plan view of a pressure damping air capacitor block to be connected between the sensor and pump of FIG. 1; and
FIG. 5 is a sectional elevational view of an assembly of the pressure damping block and the air capacitor, taken along line 5-5 of FIG. 3, and line 5-5 of FIG. 4.

### DESCRIPTION OF THE PREFERRED EMBODIMENT

The monitoring of parameters such as oxygen and carbon dioxide concentrations in inspired or expired gases in a patient's airway has become of great importance with advances in medical technology, but the accuracy of the measurement of such parameters has been adversely affected by pressure fluctuations in the airway.

In accordance with an embodiment of the invention, a test gas is drawn into a sensor through an air capacitor which stores the test gas. Thereafter, a constant pressure alternate gas source is placed in fluid communication with the air capacitor, isolating the air capacitor and the sensor from any fluctuations in the pressure of the main body of the test gas while an unknown parameter of the test gas is being measured. The embodiments of the invention thus provide a simple and effective means for accurately measuring unknown parameters of the test gas notwithstanding fluctuations in the pressure of the main body of the test gas.

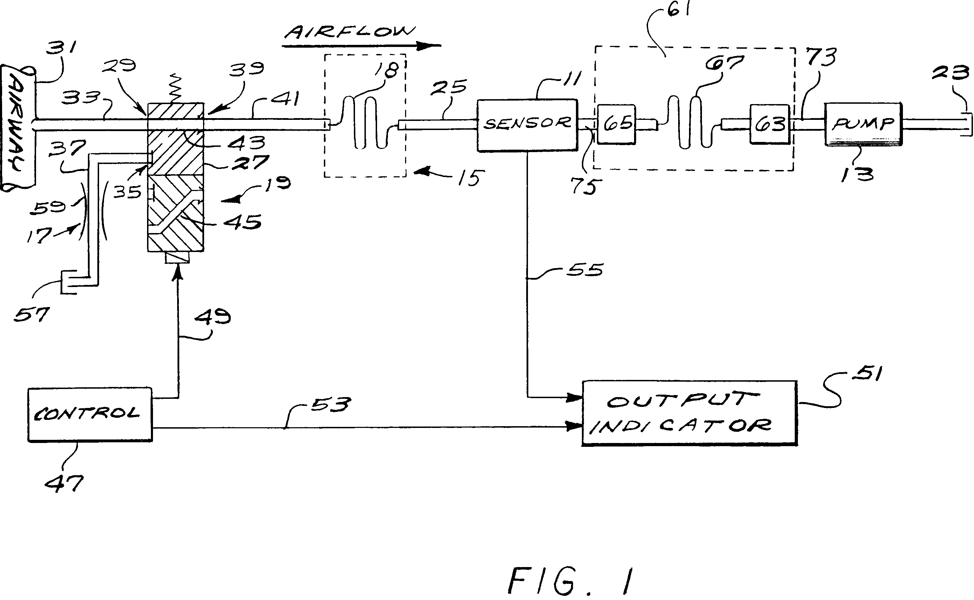

More particularly, as is shown schematically in FIGURE 1, an apparatus embodying the invention for measuring an unknown parameter of a test gas in isolation from gas pressure fluctuations comprises a sensor 11 operative to measure the unknown parameter, a pump 13 downstream from the sensor 11, an air capacitor 15 upstream from the sensor 11, a constant pressure alternate gas source generally designated 17, and fluid flow control means generally designated 19.

The pump 13 is in fluid communication with the sensor 11 through fluid flow lines 75, 73 cavities 63 and 65 and channel 67. The pump 13 draws the test gas into the sensor 11 for measurement of the unknown parameter. The gas ultimately flows out of the sensor 11 into the pump 13 and is then discarded, for example, through an exhaust vent 23.

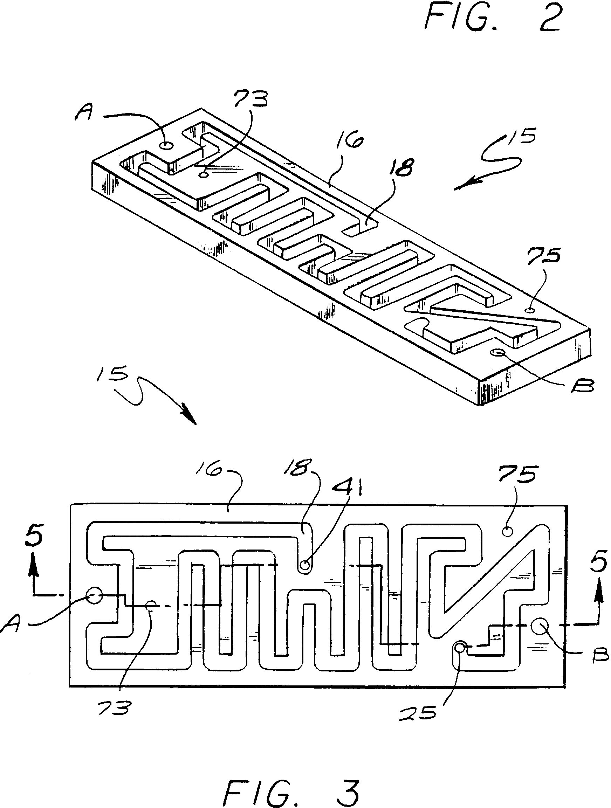

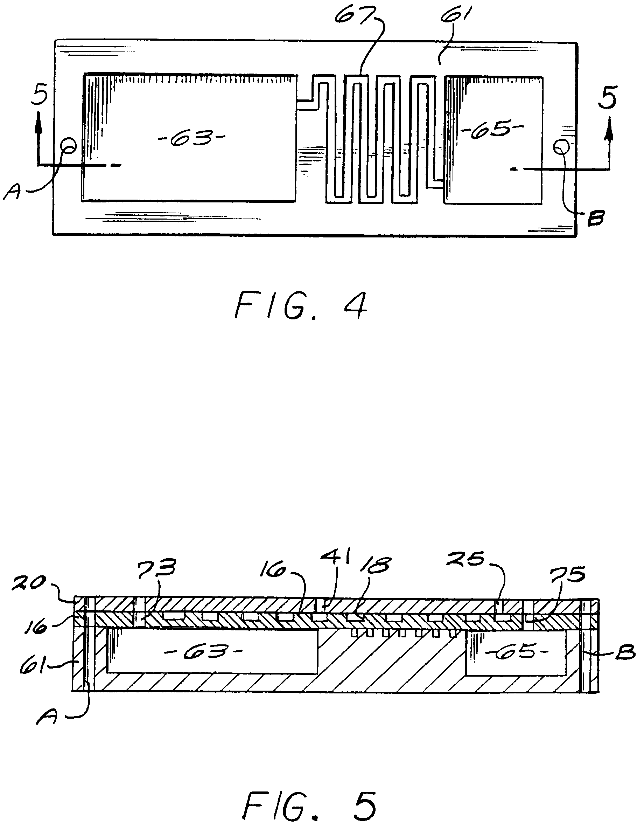

The air capacitor 15 in a currently preferred embodiment comprises a block 16 shown in Figures 2 and 3 having an enclosed channel 18 which is in fluid communication with the sensor 11 as shown by a fluid flow line 25 and stores a quantity of gas. The air capacitor channel 18 preferably comprises an elongated channel or chamber having a length-to-diameter ratio much larger than unity. For example, a channel having a length of about 75 centimeters and a diameter of about 0.5 centimeters has been found to be satisfactory. The air capacitor 15 is typically sealed by a cover 20 shown in Figure 5 to form the enclosed channel. Alternatively, the air capacitor may be formed by an appropriate length and diameter tubing which can be connected and function in a manner similar to the block form of the air capacitor.

The fluid flow control means 19 opens a first fluid flow path for the test gas into the air capacitor 15 for a sufficient time to enable the pump 13 to draw the test gas through the air capacitor 15 into the sensor 11 and thereby to fill the air capacitor 15 with said gas. Then the flow control means 19 closes the first fluid flow path and opens a second fluid flow path between the air capacitor 15 and the alternate gas source 17 and thereby isolates the sensor 11 from any fluctuations in the pressure of the test gas.

The air capacitor 15 prevents any substantial mixing of the alternate gas with the stored test gas for a sufficient time to enable the sensor 11 to measure the parameter of the test gas under constant pressure conditions without degrading the quality of the measurement. The pressure isolation provided by the air capacitor 15 permits the test gas to flow continuously through the sensor 11 during the measurement process while preventing pressure fluctuations from interfering with the accuracy of the measurement.

The fluid flow control means 19 preferably comprises a valve such as a solenoid valve 27 having a first inlet 29 in fluid communication with a source such as an airway 31 of the test gas as indicated by a fluid flow line 33, a second inlet 35 in fluid communication with the alternate gas source 17 as indicated by a fluid flow line 37, and an outlet 39 in fluid communication with the air capacitor 15 as indicated by a fluid flow line 41. The valve 27 opens the first fluid flow path by connecting the outlet 39 to the first inlet 29 as indicated by a first valve connection 43 and ovens the second flow path by connecting the outlet 39 to the second inlet 35 as indicated by a second valve connection 45.

The fluid flow control means 19 preferably also includes timing control means 47 such as a microprocessor or the like which selectively applies power to the solenoid valve 27 as indicated by a control line 49 to control the flow of gases into the air capacitor 15. The timing control means 47 causes the valve 27 to keep the first fluid flow path open long enough to fill the sensor 11 and the air capacitor 15 with the test gas, then causes the valve 27 to close the first fluid flow path and to open the second fluid flow path to isolate the air capacitor 15 and the sensor 11 from any pressure fluctuations in the source of the test gas.

The timing control means 47 also controls an output indicator 51 or the like as indicated by a control line 53. The sensor 11 provides,a parameter measurement signal to the output indicator 51 as indicated by a control line 55, and the timing control means 47 ensures that an output value is provided only during the time that the pressure of the test gas in the sensor 11 is being held constant. Preferably, the output value is delayed after the second fluid flow path has been opened just long enough for the pressure in the air capacitor 15 and the sensor 11 to stabilize.

In applications requiring frequent monitoring of an unknown parameter of the test gas, the timing control means 47 switches back and forth between the two fluid flow paths, the first path being kept open long enough to ensure that the air capacitor 15 and the sensor 11 are filled with the test gas and the second path being kept open long enough to ensure that pressures have stabilized before measurements are taken.

The alternate gas source 17 preferably comprises access to the ambient atmosphere, although other sources may be used. The second inlet 35 of the valve 27 is in fluid communication with the ambient atmosphere through the fluid flow line 37 and a vent 57. A flow constrictor 59 in the flow line 37 is preferably used to maintain constant pressure from the alternate gas source 17. In a preferred embodiment the test gas comprises inspired gas in the airway 31 of a patient respirator (not shown).

As shown in FIGS. 4 and 5, in an alternative embodiment, in order to damp out pressure fluctuations generated by the pump, the portion of the line 21 between the sensor and the pump may include a dual air capacitor block 61 which includes first and second cavities 63 and 65 and an elongated constrictor channel 67 establishing a resistor fluid flow path between the cavities 63 and 65, the channel 67 also having a length-to-diameter ratio much larger than unity.

The cavities 63 and 65 and the constrictor channel 67 are formed, for example, in an upper surface of the block 61. The block 61 may be covered by the bottom of the air capacitor block 16 and solvent bonded together to form a gas-tight seal with the adjacent cavities 63 and 65 and the channel 67 to constrain any gas flowing between the cavities 63 and 65 to flow through the channel 67. Holes A and B through the air capacitor block 16 and the block 61 may also be provided to accommodate bolts or screws for securing the two blocks together. Fluid communication for a gas to enter and exit the cavities 63 and 65 is provided by means of orifices 73 and 75, respectively, extending through the block 16 and the cover 20. The block 16 and the cover 20 may be fabricated from various materials. Acrylic plastic or the like has been found to give satisfactory results.

A method of measuring an unknown parameter of a test gas in isolation from gas pressure fluctuations by means of apparatus of the kind described above includes the steps of: opening a first fluid flow path far the test gas into an air capacitor such as the capacitor 15; drawing the test gas through the air capacitor and into a sensor such as the sensor 11 for measurement of the unknown parameter, thereby filling the air capacitor with the test gas; closing the first fluid flow path; opening a second fluid-flow path between a constant pressure alternate gas source such as the source 17 and the air capacitor and thereby isolating the sensor from any fluctuations in the pressure of the test gas; and measuring the unknown parameter of the test gas under constant pressure conditions before any substantial mixing of the alternate-gas with the test gas stored in the air capacitor occurs. In one embodiment the step of opening a first fluid flow path comprises opening a fluid flow path between an airway such as the airway 31 of a patient respirator and the air capacitor.

From the foregoing it will be appreciated that the method and apparatus described provide a way to accurately and reliably measure an unknown parameter of a gas which is subject to pressure fluctuations by storing the gas in an air capacitor and then isolating the air capacitor from the pressure fluctuations for a sufficient time to permit measurement of the unknown parameter. The apparatus and method may be used, for example, to accomplish the measurement of such parameters as oxygen and carbon dioxide concentration in inspired or expired gases in an airway of a patient respirator. This is faciltated by the fact that the apparatus is relatively simple and robust and does not interfere with the basic function of the respirator.

## Claims

1. A system for measuring a parameter of a test gas, the system including a source of the test gas, a sensor in fluid communication with the source of the test gas operative to measure the parameter of the test gas, and pump means in fluid communication with the sensor to draw the test gas from the source of the test gas into the sensor, an improvement for isolating the sensor from any fluctuations in the pressure of the test gas, characterized by:
an air capacitor upstream from the sensor in fluid communication therewith and operative to store a quantity of gas;
a source of an alternate gas having an essentially constant pressure;
fluid flow control means having a first fluid flow path connectable between said air capacitor and said source of the test gas, and a second fluid flow path connectable between said air capacitor and said source of alternate gas, the fluid flow control means being operative to open the first fluid flow path for the test gas into the air capacitor, to thereafter close said first flow path and to open the second fluid flow path between the air capacitor and the alternate source and to thereby isolate the air capacitor and the sensor from any fluctuations in the pressure of the test gas;
indicator means in electrical communication with said sensor for providing an output signal representing the measure of the unknown parameter; and
timing control means in electrical communication with said fluid flow control means and said indicator means for controlling said indicator means to provide said output signal only when the pressure of the test gas in the sensor is held constant.

2. The system according to claim 1, wherein there is further provided means for damping fluctuations in pressure in said test gas at said sensor due to said pump means downstream from said sensor, said means for damping including a first enlarged chamber connected for fluid communication with said pump means, a second enlarged chamber connected for fluid communication with said sensor, and an elongated constrictor channel for establishing a resistor fluid flow path connected for fluid communication between said first and second enlarged chambers.

3. The system according to claim 1 or claim 2, wherein the test gas is inspiration gas provided to a patient, and the fluid flow control means comprises a valve having a first inlet in fluid communication with said source of the test gas through a first flow path, a second inlet in fluid communication with the alternate gas source through a second flow path and an outlet in fluid communication with the air capacitor, the valve being operative to open the first flow path by connecting the outlet to the first inlet and to open the second flow path by connecting the outlet to the second inlet.

4. The system according to any preceding claim, wherein the alternate gas source comprises the ambient atmosphere.

5. The system according to any preceding claim, wherein the test gas is inspiration gas provided to a patient, and the source of the test gas comprises an airway of a patient respirator.

6. The system according to any preceding claim, wherein the air capacitor comprises an elongated chamber characterized by a length-to-diameter ratio larger than unity.

7. The system according to Claim 6, wherein the chamber has a length of approximately 75 centimeters and a diameter of approximately 0.5 centimeters.

8. The system according to any preceding claim, wherein the air capacitor comprises a block of material defining an elongated channel establishing a fluid flow path having a length-to-diameter ratio larger than unity.

9. The system according to claim 2 or according to any of claims 3 to 8 when dependant on claim 2, wherein said first and second enlarged chambers of said constrictor channel of said damping means are formed in a top surface of a first block member covered by a bottom surface of a second block member, and said air capacitor is formed in a top surface of said second block member covered by a third block member.

10. The system according to claim 9, wherein said second block member includes a first pair of orifices therethrough in fluid communication with said first and second enlarged chambers, said third block member includes a second pair of orifices therethrough, said first and second pairs of orifices being aligned to provide a first flow path to said first enlarged chamber and a second flow path to said second enlarged chamber, and said third block member includes a third pair of orifices therethrough to provide a third and fourth flow path to said air capacitor.

11. A method of measuring an unknown parameter of a test gas in isoloation from gas pressure fluctuations comprising the steps of opening a first fluid flow path for the test gas into an air capacitor; drawing the test gas through the air capacitor and into a sensor for measurement of the unknown parameter, thereby filling the air capacitor with the test gas; closing the first fluid flow path; opening a second fluid flow path between a constant pressure alternate gas source and the air capacitor and thereby isolating the sensor from any fluctuations in the pressure of the test gas; and measuring the unknown parameter of the test gas under constant pressure conditions before any substantial mixing of the alternate gas with the test gas stored in the air capacitor occurs.

12. The method according to claim 11, wherein the step of opening a first fluid flow path comprises opening a fluid flow path between an airway of a patient respirator and the air capacitor.
